# EUROPÄISCHE PATENTSCHRIFT

(11) **EP 0 863 133 B1**
(45) Veröffentlichungstag und Bekanntmachung des Hinweises auf die Patenterteilung: **26.09.2001**
(21) Anmeldenummer: 98103843.3
(22) Anmeldetag: 04.03.1998
(51) Int. Cl.: C07C 311/49, C07C 311/32, C07C 303/36, C07D 285/18

(54) **Verfahren zur Herstellung von 2-Amino-ethansulfonylazid-säureadditionssalzen, 2-Aminoethansulfonylazid-hydrochlorid sowie dessen Verwendung**
Process for the preparation of 2-amino-ethanesulfonylazide acid additon salts, 2-amino-ethanesulfonylazide hydrochloride as well as its use
Procédé pour la préparation de sels d'addition d'acide de 2-amino-éthanesulfonylazide, l'hydrochlorure de 2-amino-éthanesulfonylazide ainsi que son utilisation

(30) Priorität: 04.03.1997 DE 19708782
(43) Veröffentlichungstag der Anmeldung: 09.09.1998
(73) Patentinhaber: HERDEIS, Claus, 80809 München (DE); Weis, Christian Edwin, 97297 Waldbüttelbrunn (DE)
(72) Erfinder: HERDEIS, Claus, 80809 München (DE); Weis, Christian Edwin, 97297 Waldbüttelbrunn (DE)
(74) Vertreter: Andrae, Steffen, Dr.

(56) Entgegenhaltungen:
- CH-A- 482 713
- DE-C- 19 515 976
- W. KOLLER ET AL: TETRAHEDRON LETT., Bd. 24, Nr. 21, 1983, Seiten 2131-2134, XP002068729

## Beschreibung

Die vorliegende Verbindung betrifft ein Verfahren zur Herstellung von 2-Aminoethansulfonsäureazid-säureadditionssalzen, 2-Aminoethansulfonylazid-hydrochlorid sowie dessen Verwendung gemäß den Patentansprüchen.

2-Aminoethansulfonylazid-hydrochlorid stellt ein neues Zwischenprodukt zur Herstellung von Taurinamid bzw. dem daraus auf an sich bekannte Weise herstellbaren Taurolidin oder Taurultam dar. Durch die Bereitstellung von 2-Aminoethansulfonylazid-hydrochlorid und dessen nachfolgende glatte Umsetzung durch katalytische Hydrierung zu Taurinamid ist es möglich, ein verbessertes Herstellungsverfahren zur Herstellung von Taurinamid bzw. Taurolidin oder Taurultam zu schaffen.

Das neue Herstellungsverfahren, das über das Zwischenprodukt 2-Aminoethansulfonylazid-hydrochlorid verläuft, ermöglicht auch die Herstellung von Taurinamid bzw. Taurolidin oder Taurultam, ausgehend von einem anderen Ausgangsprodukt als bisher üblich, nämlich von dem zu relativ günstigen Kosten im Handel erhältlichen 2-Aminoethanthiol (Cysteamin). Anstelle von Cysteamin kann als Ausgangsprodukt auch das entsprechende Disulfid (Cystamin; Bis-(2-aminoethyl)-disulfid) verwendet werden, das ebenfalls als Handelsprodukt erhältlich ist. Beide Ausgangsprodukte, Cysteamin und Cystamin, werden insbesondere in Form von Säureadditionssalzen, nämlich Halogenwasserstoff-additionssalzen, insbesondere der Hydrochloride, bezogen bzw. eingesetzt.

Taurolidin und Taurultam sind Verbindungen der Formeln:

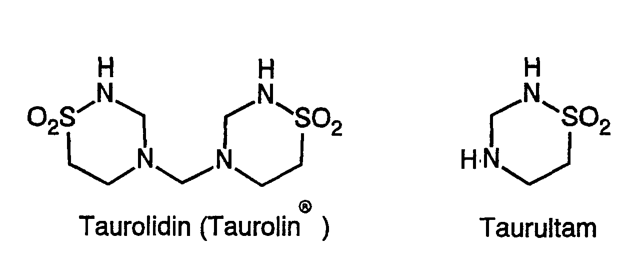

Taurolidin ist eine antibakteriell und antiendotoxisch wirksame Substanz, die insbesondere in Form einer antiseptischen Spüllösung in der Chirurgie zum Auswaschen des Bauchraumes verwendet wird und gegenüber anderen antiseptisch wirksamen Substanzen den Vorteil aufweist, daß sie auch freigesetzte Toxine unschädlich macht und damit zur Verhinderung eines septischen Schocks beitragen kann. Taurolidin wird in Form von Lösungen für den Krankenhausbedarf im Handel angeboten (Taurolin® -Lösung).

Eine weitere Verwendungsmöglichkeit für Taurolidin ist in der Patentanmeldung DE 4239206 A1 beschrieben, die insbesondere den Einsatz in Haarshampoos betrifft, wo es dazu dient, die Formbarkeit der Haare zu verbessern.

Taurultam kann durch Hydrierung aus Taurolidin erhalten werden und hat ähnliche Eigenschaften wie dieses, kann dieses somit bei vielen Anwendungen ersetzen. Die Verbindung mit ihren physikalischen und spektroskopischen Daten ist beschrieben in Hagers Handbuch der Pharmazeutischen Praxis, 5. Auflage, Bd.9, S. 780.

Bei der bekannten Herstellung von Taurolidin wird als wesentliche Zwischenstufe die Verbindung Taurinamid bzw. deren Säureadditionssalz durchlaufen. Letzteres wird im letzten Schritt bei der bekannten Herstellung von Taurolidin unter basischen Bedingungen mit 1,5 Äquivalenten Formaldehyd umgesetzt. Diese Herstellung ist beschrieben in der Schweizer Patentschrift 482 713, insbesondere in Beispiel 4. Weitere Beschreibungen finden sich z.B. in Hagers Handbuch der Pharmazeutischen Praxis, 5. Auflage, Bd.9, S.779-780 und in der Monographie von Brückner, W.L.; Pfirrmann, R.W., Taurolin® (1985), Verlag Urban und Schwarzenberg, München, Wien, Baltimore. Bei den bisherigen Verfahren zur Herstellung des benötigten Taurinamids bzw. dessen Säureadditionssalzen wird ausgegangen von 2-Aminoethansulfonsäure (Taurin). Die bekannten Verfahren (vgl. DE 3 620 667 A1 oder US-A-2 184 279) zur Herstellung von Taurinamid, ausgehend von Taurin, sind mehrstufige Verfahren mit einem relativ hohen Chemikalienbedarf und einer gleichzeitigen Erzeugung von erheblichen Mengen an unerwünschten Nebenprodukten, die entsorgt werden müssen. Bei derartigen Verfahren muß die Aminogruppe des Taurins zuerst geschützt werden (beispielsweise durch Umsetzung mit Phthalsäureanhydrid oder Trifluoressigsäurechlorid), damit die Sulfonsäuregruppe anschließend mit Chlorierungsmitteln (z.B. Thionylchlorid, Phosphorylchlorid) in eine Sulfonylchlorid-gruppe übergeführt werden kann. Aus dem nach der Chlorierung erhaltenen Sulfonylchlorid wird durch Umsetzung mit Ammoniak dann das entsprechende Sulfonamid hergestellt, und die Schutzgruppe für die 2-Aminogruppe muß wieder abgespalten werden. Das Taurinamid wird schließlich in Form seines Hydrochlorids isoliert.

Es ist außerdem bekannt, Taurinamid ausgehend von dem ungesättigten Sulfonamid der Formel CH₂=CHSO₂NH₂ oder dem entsprechenden ungesättigten Sulfonylhalogenid CH₂=CHSO₂Hal (Hal=Halogenatom, insbesondere Cl) herzustellen.

Verfahren, die von Vinylsulfonamiden ausgehen, sind mit dem Nachteil behaftet, daß sich unerwünschte Nebenprodukte bilden und die Vinylsulfonsäurederivate relativ instabile Verbindungen darstellen, was die Verfahrensführung erschwert. Außerdem stellen Vinylsulfonsäurederivate relativ teure Ausgangssubstanzen dar, deren Kosten sich auf das Endprodukt auswirken.

Die eingangs beschriebenen, von Taurin ausgehenden Verfahren sind im Hinblick auf die benötigten Reagenzien und Verfahrensnebenprodukte unter Gesichtspunkten der Umweltverträglichkeit nachteilig und als Mehrstufenverfahren aufwendig und auch im Hinblick auf die Gesamtausbeute nicht befriedigend. So entspricht bei dem derzeit in der Praxis angewandten fünfstufigen Verfahren zur Herstellung von Taurinamid die Menge der unerwünschten Nebenprodukte etwa der Menge des hergestellten Taurinamids.

Aus der deutschen Patentschrift DE 195 15 976 C1 ist ein Verfahren bekannt geworden, bei dem man zur Herstellung von Taurinamid bzw. Taurolidin einen grundsätzlich anderen Weg beschreitet, indem man ausgehend von insbesondere β-Chlorethansulfonylchlorid durch Umsetzung mit einem geeigneten Azid ein Diazid, β-Azidoethansulfonylazid, herstellt, das durch katalytische Hydrierung in Taurinamid überführt werden kann, das dann seinerseits auf an sich bekannte Weise zu Taurolidin weiterverarbeitet werden kann.

Das Verfahren gemäß DE 195 15 976 C1 hat gegenüber den herkömmlichen Verfahren den Vorteil, daß die Herstellung von Taurolidin ohne Bildung nennenswerter Mengen an unerwünschten Nebenprodukten und nahezu quantitativ erfolgt.

Ein gewisser Nachteil des Verfahrens gemäß DE 195 15 976 C1 ist jedoch die potentielle Explosionsneigung von *β*-Azidoethansulfonylazid. Ferner ist das genannte Verfahren auch im Hinblick auf die Kosten für die verwendeten Ausgangsverbindungen und den Lösungsmittelbedarf noch verbesserungsfähig.

Es ist daher eine Aufgabe der vorliegenden Erfindung, eine neue Verbindung bereitzustellen, die unter Beibehaltung wesentlicher Vorteile der Azidverbindung gemäß DE 195 15 976 C1 sicherer zu handhaben ist und deren Verwendung als Zwischenprodukt die Schaffung eines neuen Verfahrens zur Herstellung von Taurinamid ermöglicht, das gegenüber den bekannten Verfahren geringere Betriebsrisiken aufweist und von kostengünstiger erhältlichen Ausgangsverbindungen ausgeht, so daß letztlich insgesamt ein verbessertes Verfahren zur Herstellung von Taurolidin bzw. Taurultam geschaffen wird.

Diese Aufgabe wird einerseits durch die neue Verbindung 2-Aminoethansulfonylazid-hydrochlorid gelöst.

Die Aufgabe wird andererseits dadurch gelöst, daß eine über die neue Schlüsselverbindung 2-Aminoethansulfonylazid-hydrochlorid als Zwischenstufe verlaufende Taurinamidsynthese bzw. Taurolidin- oder Taurultamsynthese geschaffen wird.

Ein besonderer Aspekt der vorliegenden Erfindung liegt darin, daß die Herstellung von Taurolidin, ausgehend von einer neuen Ausgangsverbindung ermöglicht wird, nämlich unter Verwendung des relativ kostengünstig im Handel erhältlichen 2-Aminoethanthiols (Cysteamin) bzw. des entsprechenden Disulfids (Cystamin) bzw. von deren Säureadditionssalzen, insbesondere der Hydrochloride.

Das neue Verfahren unterscheidet sich von dem Verfahren gemäß DE 195 15 976 C1 im wesentlichen dadurch, daß man von Anfang an mit Verbindungen arbeitet, die eine zur Salzbildung befähigte Aminogruppe in der 2-Stellung (β-Stellung) enthalten. Die Aminogruppe kann während der Umsetzungsschritte des Verfahrens durch einfache Salzbildung mit einer Säure, insbesondere einer starken einbasigen Mineralsäure und dabei vorzugsweise einer Halogenwasserstoffsäure, insbesondere mit Chlorwasserstoff, geschützt werden und ermöglicht aufgrund der Löslichkeit der salzartigen Verbindungen die Verwendung wäßriger Reaktionsmedien. So kann nach dem erfindungsgemäßen Verfahren die Herstellung von Taurolidin überraschender Weise auch im Sinne einer Eintopfsynthese in Wasser als Reaktionsmedium durchgeführt werden, wie nachfolgend noch näher erläutert wird.

2-Aminoethansulfonylazid wird erfindungsgemäß in Form eines Säureadditionssalzes, nämlich des Hydrochlorids, nach einem Verfahren hergestellt, bei dem in der der Einführung der Azidgruppe vorausgehenden Verfahrensstufe eine Verbindung der Formel (I)

HX.H₂NCH₂CH₂SO₂X'

durchlaufen wird, wobei in dieser Verbindung X und X' gleich und verschieden sein können und vorzugsweise jeweils für ein Halogenatom, insbesondere Chlor oder Brom, stehen. Die genannten Verbindungen der Formel (I), die Säureadditionssalze von β-Aminoethansulfonylhalogeniden sind, können auch als Säureadditionssalze von Taurylhalogeniden bezeichnet werden, beispielsweise im Falle von sowohl X als auch X' Chlor als Taurylchlorid-hydrochlorid.

Bei diesen Verbindungen der Formel (I) kann der an die SO₂-Gruppe gebundene Halogenrest durch nucleophile Substitution in glatter Reaktion durch eine Azidgruppe ersetzt werden, die dann nachfolgend durch katalytische Hydrierung, ähnlich wie bei dem Verfahren gemäß DE 195 15 976 C1, in eine Amidgruppe umgewandelt werden kann.

Es ist grundsätzlich bekannt, daß Säureadditionssalze von β-Aminoalkansulfonylhalogeniden durch oxidative Halogenierung von 2-Aminoalkanthiolen oder -disulfiden erhältlich sind (vgl. z.B. Tetrahedron Letters, Vol. 24, Nr. 21, S. 2131-2134, 1983 und die darin zitierte Literatur; sowie Tetrahedron Letters Nr. 3, S. 213-216, 1972). Es ist jedoch nicht bekannt, Säureadditionssalze von 2-Aminoalkansulfonylhalogeniden zu Taurinamid umzusetzen. Versucht man beispielsweise, das an die Sulfonylgruppe gebundene Halogenatom durch Umsetzung mit Ammoniak durch eine Aminogruppe zu ersetzen, kommt es aufgrund der Basizität des Ammoniaks dazu, daß die 2-Aminogruppe aus ihrem Salz freigesetzt wird und die neutrale Verbindung unter Ringschluß zu der entsprechenden Sultamverbindung reagiert (a.a.O.).

Indem man im beanspruchten Verfahren z.B. das 2-Aminoethansulfonylchlorid-hydrochlorid in einer nucleophilen Substitutionsreaktion unter Verwendung eines geeigneten Azids in einem geeigneten polaren Lösungsmittel in die erfindungsgemäße Verbindung 2-Aminoethansulfonylazid-hydrochlorid umwandelt, wird die Reaktion zur Sultamverbindung vermieden, und das Taurinamid-hydrochlorid kann anschließend durch einfache katalytische Hydrierung aus dem genannten Azid-hydrochlorid hergestellt werden.

Grundsätzlich können für die Umsetzung des 2-Aminoethansulfonylchlorid-hydrochlorids mit einem geeigneten Azid als Lösungsmittel insbesondere Wasser, ggf. jedoch auch organische Lösungsmittel wie dipolar aprotische Lösungsmittel, substituierte und unsubstituierte Amide, cyclische oder acyclische Ether verwendet werden. Arbeitet man in Wasser, wird eine quantitative Umsetzung von 2-Aminoethansulfonylchlorid-hydrochlorid zu 2-Aminoethansulfonylazid-hydrochlorid erreicht. Bei Raumtemperatur ist die Reaktion nach 15 Minuten quantitativ abgeschlossen. Wasser ist daher das bevorzugte Reaktionssmedium für die erfindungsgemäße Herstellung von 2-Aminoethansulfonylazid-hydrochlorid. Ein Arbeiten in anderen Lösungsmitteln mit einem vergleichbaren Lösungsverhalten, insbesondere Lösungsmitteln hoher Polarität, soll jedoch nicht ausgeschlossen sein.

Die neue Verbindung 2-Aminoethansulfonylazid-hydrochlorid. stellt, wenn sie nach dem nachfolgend beschriebenen Verfahren hergestellt wird, eine farblose kristalline Verbindung dar (Schmelzpunkt: 155°C, umkristallisiert aus absolutem Ethanol) . Sie kann bei Raumtemperatur sicher gehandhabt werden, weist aber wie andere Sulfonylazide bei höheren Temperaturen (> 200°) eine Neigung zur Zersetzung auf. Die Verbindung ist nicht schlagempfindlich, was einen erheblichen Vorteil gegenüber 2-Azidoethansulfonsäureazid darstellt.

Es hat sich gezeigt, daß 2-Aminoethansulfonylazid-hydrochlorid glatt zu dem gewünschten Taurinamid bzw. dessen Säureadditionssalz reduziert werden kann. Vorzugsweise führt man die Reaktion als katalytische Hydrierung in Anwesenheit eines Metallkatalysators (vorzugsweise Palladium oder Platin auf einem geeigneten Träger wie Aktivkohle) in einem geeigneten Lösungsmittel durch. Das Lösungsmittel wird im Hinblick auf seine Eignung als Hydrierungslösungsmittel sowie außerdem im Hinblick auf seine leichte und saubere Abtrennung vom Endprodukt gewählt. Als besonders geeignet haben sich Wasser oder die niedrigen Alkanole CₙH₂ₙ₊₁OH erwiesen.

Das durch die Hydrierung des erfindungsgemäßen Azid-hydrochlorids hergestellte Taurinamid-hydrochlorid kann isoliert und auf bekannte Weise gemäß dem Verfahren aus der Schweizer Patentschrift 482 713 bzw. wie in Beispiel 2 unter b) der DE 195 15 976 C1 beschrieben mit Formaldehyd unter basischen Bedingungen zu Taurolidin umgesetzt werden. Dieses kann gewünschtenfalls auch noch in Taurultam umgewandelt werden.

Ein besonderer Vorteil des erfindungsgemäßen Verfahrens auch gegenüber dem Verfahren gemäß DE 195 15 976 C1 besteht jedoch darin, daß alle Reaktionsschritte von der Einführung der Azidgruppe bis zur Herstellung des Endprodukts Taurolidin in einem wäßrigen Medium durchgeführt werden können, ohne daß es erforderlich ist, die hergestellten Zwischenstufen zu isolieren. Wie das Verfahren gemäß DE 195 15 976 C1 hat das erfindungsgemäße Verfahren gegenüber den sonstigen Verfahren des Standes der Technik den erheblichen Vorteil, daß dann, wenn man mit den Chloriden bzw. Hydrochloriden und NaN₃ als Azid arbeitet, als einziges Nebenprodukt Kochsalz anfällt. Der Katalysator ist regenerierbar, es kann in Wasser gearbeitet werden, und die Umsetzungen verlaufen nahezu quantitativ.

Ein weiterer wesentlicher Vorteil des erfindungsgemäßen Verfahrens besteht darin, daß von den kostengünstig im Handel erhältlichen Verbindungen Cysteamin bzw. Cysteamin-hydrochlorid (2-Aminoethanthiol-hydrochlorid) oder ggf. Cystamin bzw. Cystamin-hydrochlorid ausgegangen werden kann, wodurch das erfindungsgemäße Verfahren auch auf der Kostenebene gegenüber dem Verfahren gemäß DE 195 15 976 C1 vorteilhaft abschneidet.

Nachfolgend werden die verschiedenen Aspekte der vorliegenden Erfindung anhand von Beispielen noch näher erläutert.

### Beispiel 1

a) Herstellung von 2-Aminoethansulfonylchlorid-hydrochlorid (Taurylchlorid-hydrochlorid) aus Cysteamin-hydrochlorid
   In einem Dreihalskolben mit einem mechanischen Rührer, einem Gaseinleitungsrohr und einem Innenthermometer wurde Cysteamin-hydrochlorid (5,74 g; 0,05 mol) in einer Mischung aus Tetrachlormethan/Ethanol (50 ml/6ml) suspendiert. Unter Verwendung einer Außenkühlung mit einer Eis/Wasser-Mischung wurde die Reaktionsmischung auf ca. 5°C abgekühlt, wonach unter Rühren Chlorgas eingeleitet wurde, das sofort mit dem Inhalt des Reaktionsgefäßes reagierte. Nach einer ca. 15-minütigen Chlorgaseinleitung nahm die Suspension ein cremiges Aussehen an, und die Reaktionstemperatur erhöhte sich dabei auf etwa 20 bis 30°C. Die bei der Umsetzung entstehenden Gase (HCl, Ethylchlorid) wurden durch Einleiten in eine Absorptionsflüssigkeit gebunden. Die Chlorgas-Einleitung wurde so lange fortgesetzt, bis die Reaktionsmischung eine stabile Gelbfärbung angenommen hatte (nach ca. 45 min).
   In der Reaktionsmischung war eine farblose Substanz suspendiert, und eine ¹H-NMR-spektroskopische Untersuchung (in D₂O) zeigte, daß die Ausgangsverbindung quantitativ mit einer Selektivität von mehr als 95% abreagiert hatte.
   Über eine Glasfilternutsche wurde das in der Reaktionsmischung suspendierte Taurylchlorid-hydrochlorid vom flüssigen Reaktionsmedium abgetrennt. Ein Waschen, z.B. mit einem organischen Lösungsmittel, ist möglich, jedoch nicht erforderlich. Nach dem Trocknen des Produkts im Vakuum bei Raumtemperatur wurde das Produkt der Weiterverarbeitung zugeführt.
   In einem verschlossenen Gefäß kann das Taurylchlorid-hydrochlorid aufbewahrt werden, ohne zu hydrolysieren. Die Verbindung ist thermisch stabil.
   Bei einer Ansatzvergrößerung ausgehend von 1 mol (113,6 g) Cysteamin-hydrochlorid wurde Taurylchlorid-hydrochlorid in quantitativer Ausbeute erhalten.
   **Schmelzpunkt** (Rohmaterial) 155-158°C
   **IR** (KBr): *v* (cm⁻¹) = 3300-2700, 22650, 2450, 2000-1900, 1600, 1550, 1515, 1460, 1450, 1410, 1400, 1380-1390, 1280, 1170, 1160, 1110, 1080, 1040, 950, 870, 840, 760, 700.
   ^{**1**}**H-NMR** (D₂O, 200 MHz): δ (ppm) = 3.71 (t, ³J = 6.2 Hz, 2H), 4.41 (t, ³J = 6.2 Hz, 2H).
   ^{**13**}**C-NMR** (D₂O, 50,3 MHz): δ (ppm) = 61.34 (H₃N-CH₂), 35.1 (CH₂SO₂Cl).
b) Herstellung von 2-Aminoethansulfonylazid-hydrochlorid (Taurylazid-hydrochlorid) aus 2-Aminoethansulfonylchlorid-hydrochlorid (Taurylchlorid-hydrochlorid)
   Taurylchlorid-hydrochlorid und Natriumazid wurden im Verhältnis 1:1 in Wasser miteinander umgesetzt. Dazu wurde die berechnete stöchiometrische Menge an Natriumazid in Wasser gelöst, und die Lösung wurde auf 5°C abgekühlt. Unter Rühren wurde Taurylchlorid-hydrochlorid zugegeben, und die Reaktionsmischung wurde 15 min gerührt. Durch Abziehen des Wassers erhielt man eine Feststoffmischung aus kristallinem Natriumchlorid und Taurylazid-hydrochlorid. Durch Umkristallisation aus absolutem Ethanol erhält man Taurylazid-hydrochlorid frei von NaCl.
   **Schmelzpunkt:** 155°C (aus Ethanol).
   **IR** (KBr): *ν* (cm⁻¹) = 3100, 2990, 2820-2920, 2120 (CN), 1590, 1560, 1500, 1450, 1390, 1360, 1340, 1230, 1200, 1180, 1120, 100, 1040, 950, 870, 840, 745, 700.
   ^{**1**}**H-NMR** (D₂O, 200 MHz): δ (ppm) = 3.71 (t, ³J = 6.4 Hz, 2H), 4.18 (t, ³J = 6.4 Hz, 2H).
   ^{**13**}**C-NMR** (D₂O, 50.3 MHz): δ (ppm) = 52.61 (H₃N-CH₂), 34.63 (CH₂SO₂N₃).

### Beispiel 2 (erfindungsgemäße Verwendung)

### Hydrierung von 2-Aminoethansulfonylazid-hydrochlorid zu Taurinamid-hydrochlorid

Das gemäß Beispiel 1 erhaltene Taurylazid-hydrochlorid-Produkt wurde in Wasser gelöst, mit einem Hydrierungskatalysator (Palladium auf Kohle) versetzt, und unter Einleiten von Wasserstoff mit einem Druck von 40 bar wurde die eingesetzte Verbindung 3 h lang hydriert. Durch eine Überwachung der Hydrierung zeigte sich, daß die Hydrierung nach 3 h quantitativ abgelaufen war (¹H- und ¹³C-NMR-Kontrollspektren zeigten nur noch Produktsignale). Der Katalysator wurde daraufhin von der Reaktionsmischung abfiltriert, und das Reaktionsmedium Wasser wurde im Vakuum entfernt. Es wurde ein klebriger farbloser Rückstand erhalten, der mit absolutem Ethanol versetzt und durch Erwärmen teilweise darin gelöst wurde. Beim Abkühlen wurde das Produkt aus der Lösung zur vollständigen Kristallisation gebracht und von der flüssigen Phase durch Abfiltrieren getrennt. Der erhaltene Filtrationsrückstand bestand aus Taurinamid-hydrochlorid und Natriumchlorid. Unter Verwendung von absolutem Ethanol konnte Taurinamid-hydrochlorid in umkristallisierter Form rein gewonnen werden.

Eine Besonderheit der Reinigung durch Umkristallisation besteht darin, daß ein Umkristallisieren unter Verwendung von Ethanol, der durch Petroletherzusätze vergällt ist, nicht möglich ist.
Ausbeute: 60-70% (nach Umkristallisation aus absolutem Ethanol)
**Schmelzpunkt:** 132-134°C (aus absolutem Ethanol)
Schmelzpunktangaben verschiedener Hersteller:
Fa. Geistlich: 130-132° (umkristallisiert);
Fa. Kali-Chemie: 132°C;
Beim Weitererhitzen kommt es bei 248-252°C zu einer Braunverfärbung und Zersetzung des Produkts.
**IR:** ν (cm⁻¹) = 3220, 3100-3200 (br), 2920, 2710, 1950, 1600, 1585, 1560, 1490, 1455, 1415, 1390, 1320, 1290, 1150, 1090, 1030, 940, 915, 880, 840, 750.
^{**1**}**H-NMR** (D₂O, 200 MHz): δ (ppm) = 3.56 (t, ³J = 6.4 Hz, 2H), 3.74 (t, ³J = 6.4 Hz, 2H).
^{**13**}**C-NMR** (D₂O, 50.3 MHz): δ (ppm) = 51.76 (H₃N-CH₂), 35.07 (CH₂SO₂N₃).

### Beispiel 3

### Herstellung von Taurolidin aus Taurylchlorid-hydrochlorid ohne Isolierung der Zwischenprodukte

Wie in Beispiel 1b) beschrieben wurde die stöchiometrisch berechnete Menge an Natriumazid in Wasser gelöst, und die Lösung wurde auf 5°C abgekühlt. Anschließend wurde eine entsprechende stöchiometrische Menge Taurylchlorid-hydrochlorid unter Rühren zugegeben, und es wurde so lange gerührt, bis eine vollständige Auflösung erreicht war (nach ca. 15 min). Die erhaltene Lösung wurde direkt mit einem Hydrierungskatalysator (Palladium auf Kohle) versetzt und, wie in Beispiel 3 beschrieben, 3 h lang bei 40 bar durch Einleiten von Wasserstoff hydriert. Nach Abschluß der 3-stündigen Hydrierung (¹H- und ¹³C-NMR-Kontrollspektren zeigten nur noch Produktsignale) wurde der Hydrierungskatalysator von der Reaktionsmischung abfiltriert. Die als Filtrat erhaltene Lösung des Taurinamidhydrochlorids wurde durch Abziehen eines Teils des Wassers auf die in der Literatur (a.a.O.) angegebene Konzentration gebracht, auf 5°C abgekühlt und mit den für die Umsetzung des Taurinamids zu Taurolidin nach den bekannten Verfahren erforderlichen Mengen an Natronlauge (30%) und Formaldehydlösung (37%) versetzt. Unter anhaltendem Rühren fällt Taurolidin nach einiger Zeit als farbloses, feinkristallines Pulver aus, das durch Absaugen über eine Glasfritte von der flüssigen Reaktionsmischung getrennt wurde. Nach einem Trocknen im Feinvakuum betrug der Schmelzpunkt des Produkts 172-175°C.

Es wurde eine 90%ige Ausbeute, bezogen auf das eingesetzte Taurylchlorid-hydrochlorid, errechnet.

### Beispiel 4

### Herstellung von Taurultam aus Taurylchlorid-hydrochlorid ohne Isolierung der Zwischenprodukte

Taurultam wird ausgehend von Taurylchlorid-hydrochlorid unter identischen Bedingungen wie in Beispiel 3 beschrieben hergestellt, wobei der einzige Unterschied darin besteht, daß man in der letzten Umsetzung eine solche Menge an Formaldehydlösung einsetzt, daß ein stöchiometrisches Verhältnis von Taurinamid-hydrochlorid zu Formaldehyd von 1:1 erhalten wird.

Die physikalischen und spektroskopischen Eigenschaften des Produkts entsprechen den der Literatur entnehmbaren Daten (vgl. Hagers Handbuch der Pharmazeutischen Praxis, 5. Auflage, Bd.9, S.780).

## Patentansprüche

1. 2-Aminoethansulfonylazid und seine Säureadditionssalze.

2. Verfahren zur Herstellung von 2-Aminoethansulfonylazidsäureadditionssalzen, **dadurch gekennzeichnet, daß** man
(i) 2-Aminoethanthiol (Cysteamin) oder Bis-(2-aminoethyl)-disulfid (Cystamin) in Form eines ihrer Säureadditionssalze auf an sich bekannte Weise durch oxidative Halogenierung in ein 2-Aminoethansulfonylhalogenid-säureadditionssalz (Taurylhalogenid-säureadditionssalz) überführt,
(ii) und das erhaltene 2-Aminoethansulfonylhalogenid-säureadditionssalz durch Umsetzung mit einem geeigneten nukleophilen Azid in ein Säureadditionssalz von 2-Aminoethansulfonylazid (Taurylazid) überführt.

3. Verfahren nach Anspruch 2, **dadurch gekennzeichnet, daß** man in Stufe (i) 2-Aminoethanthiol (Cysteamin) oder Bis-(2-aminoethyl)-disulfid (Cystamin) in Form ihrer Hydrochloride auf an sich bekannte Weise durch Umsetzung mit gasförmigem Chlor in Gegenwart von Ethanol in 2-Aminoethansulfonylchloridhydrochlorid (Taurylchlorid-hydrochlorid) überführt.

4. Verfahren nach Anspruch 2 oder 3, **dadurch gekennzeichnet, daß** man Stufe (ii) in einem polaren Lösungsmittel, insbesondere Wasser oder einem C₁-C₄-Alkanol, als Reaktionsmedium durchführt.

5. Verfahren nach Anspruch 4, **dadurch gekennzeichnet, daß** man als Azid ein Alkalimetallazid, insbesondere Natriumazid, verwendet.

6. Verfahren nach einem der Ansprüche 1 bis 5, **dadurch gekennzeichnet, daß** die Säureadditionssalze Salze einer Halogenwasserstoffsäure, insbesondere Hydrochloride, sind.

7. Verwendung von 2-Aminoethansulfonylazid, insbesondere in Form seines Hydrochlorids, oder der Produktlösung des Verfahrens gemäß Anspruch 2 zur Herstellung von 2-Aminoethansulfonylamid-hydrochlorid (Taurinamid-hydrochlorid) und Taurolidin oder Taurultam durch Reduktion.

8. Verwendung nach Anspruch 7, **dadurch gekennzeichnet, daß** man 2-Aminoethansulfonylazid-hydrochlorid oder die Produktlösung des Verfahrens gemäß Anspruch 2 durch katalytische Hydrierung in 2-Aminoethansulfonylamid-hydrochlorid überführt.

9. Verwendung nach Anspruch 7 und 8, **dadurch gekennzeichnet, daß** man die katalytische Hydrierung in einem wäßrigen Medium durchführt und nach Abtrennung des verwendeten Hydrierungskatalysators die Produktlösung, die das gebildete 2-Aminoethansulfonylamid-hydrochlorid enthält, durch Zugabe von Formaldehyd und einer Base auf an sich bekannte Weise direkt zu Taurolidin weiterverarbeitet.

10. Verwendung von 2-Aminoethanthiol (Cysteamin) oder Bis-(2-aminoethyl)-disulfid (Cystamin), ggf. in Form ihrer Säureadditionssalze, als Ausgangsverbindung zur Herstellung von Taurolidin oder Taurultam über das 2-Aminoethansulfonylazid.

## Claims

1. 2-Aminoethanesulphonylazide and its acid addition salts.

2. Process for the preparation of 2-aminoethanesulphonylazide acid addition salts, **characterized in that**
(i) 2-aminoethanethiol (cysteamine) or bis(2-aminoethyl) disulphide (cystamine) in the form of one of their acid addtion salts is converted, in a manner known per se, into a 2-aminoethanesulphonyl halide acid addition salt (tauryl halide acid addition salt) by oxidative halogenation,
(ii) and the 2-aminoethanesulphonyl halide acid addition salt obtained is converted into an acid addition salt of 2-aminoethanesulphonylazide (tauryl azide) by reaction with a suitable nucleophilic azide.

3. Process according to Claim 2, **characterized in that**, in stage (i), 2-aminoethanethiol (cysteamine) or bis(2-aminoethyl) disulphide (cystamine) in the form of their hydrochlorides are converted, in a manner known per se, by reaction with gaseous chlorine in the presence of ethanol into 2-aminoethanesulphonyl chloride hydrochloride (tauryl chloride hydrochloride).

4. Process according to Claim 2 or 3, **characterized in that** stage (ii) is carried out in a polar solvent, in particular water or a C₁-C₄-alkanol, as the reaction medium.

5. Process according to Claim 4, **characterized in that** the azide used is an alkali metal azide, in particular sodium azide.

6. Process according to any of Claims 1 to 5, **characterized in that** the acid addition salts are salts of a hydrohalic acid, in particular hydrochlorides.

7. Use of 2-aminoethanesulphonylazide, in particular in the form of its hydrochloride, or of the product solution of the process according to Claim 2, for the preparation of 2-aminoethanesulphonylamide hydrochloride (taurylamide hydrochloride) and taurolidine or taurultam by reduction.

8. Use according to Claim 7, **characterized in that** 2-aminoethanesulphonylazide hydrochloride or the product solution of the process according to Claim 2 is converted into 2-aminoethanesulphonylamide hydrochloride by catalytic hydrogenation.

9. Use according to Claims 7 and 8, **characterized in that** the catalytic hydrogenation is carried out in an aqueous medium and, after the hydrogenation catalyst used has been separated off, the product solution containing the formed 2-aminoethanesulphonylamide hydrochloride is further processed, in a manner known per se, directly into taurolidine by addition of formaldehyde and of a base.

10. Use of 2-aminoethanethiol (cysteamine) or bis(2-aminoethyl) disulphide (cystamine), optionally in the form of their acid addition salts, as a starting compound for the preparation of taurolidine or taurultam via 2-aminoethanesulphonylazide.

## Revendications

1. Azide de 2-aminoéthanesulfonyle et ses sels d'addition d'acide.

2. Procédé de préparation de sels d'addition d'acide d'azide de 2-aminoéthanesulfonyle, **caractérisé en ce que**
(i) on transforme du 2-aminoéthanethiol (cystéamine) ou du disulfure de bis(2-aminoéthyle) (cystamine), sous la forme de l'un de ses sels d'addition d'acide, d'une manière connue par halogénation par oxydation, en un sel d'addition d'acide d'halogénure de 2-aminoéthanesulfonyle (sel d'addition d'acide d'halogénure de tauryle),
(ii) et on transforme le sel d'addition d'acide d'halogénure de 2-aminoéthanesulfonyle obtenu, par réaction avec un azide nucléophile approprié, en un sel d'addition d'acide d'azide de 2-aminoéthanesulfonyle (azide de tauryle).

3. Procédé selon la revendication 2, **caractérisé en ce que**, dans l'étape (i), on transforme le 2-aminoéthanethiol (cystéamine) ou le disulfure de bis(2-aminoéthyle) (cystamine), sous la forme de leur chlorhydrate, en chlorhydrate de chlorure de 2-aminoéthanesulfonyle (chlorhydrate de chlorure de tauryle) d'une manière connue par réaction avec du chlore gazeux en présence d'éthanol.

4. Procédé selon la revendication 2 ou 3, **caractérisé en qu**'on effectue l'étape (ii) dans un solvant polaire, en particulier de l'eau ou un alcanol en C₁-C₄, en tant que milieu de réaction.

5. Procédé selon la revendication 4, **caractérisé en ce qu'**on utilise, comme azide, un azide de métal alcalin, en particulier de l'azide de sodium.

6. Procédé selon l'une des revendications 1 à 5, **caractérisé en ce que** les sels d'addition d'acide sont des sels d'un hydracide halogéné, en particulier des chlorhydrates.

7. Utilisation d'azide de 2-aminoéthanesulfonyle, en particulier sous la forme de son chlorhydrate, ou de la solution du produit du procédé selon la revendication 2, pour la préparation de chlorhydrate d'amide de 2-aminoéthanesulfonyle (chlorhydrate de taurinamide) et de taurolidine ou de taurultame par réduction.

8. Utilisation selon la revendication 7, **caractérisée en ce qu'**on transforme le chlorhydrate de 2-aminoéthanesulfonyle ou la solution du produit du procédé selon la revendication 2 en chlorhydrate d'amide de 2-aminoéthanesulfonyle par hydrogénation catalytique.

9. Utilisation selon les revendications 7 et 8, **caractérisée en ce qu'**on effectue l'hydrogénation catalytique dans un milieu aqueux, et après la séparation du catalyseur d'hydrogénation utilisé, la solution de produit qui contient le chlorhydrate d'amide de 2-aminoéthanesulfonyle formé est transformée de façon supplémentaire directement en taurolidine d'une manière connue par addition de formaldéhyde et d'une base.

10. Utilisation de 2-aminoéthanethiol (cystéamine) ou de dichlorure de bis(2-aminoéthyle) (cystamine), éventuellement sous la forme de leurs sels d'addition d'acide, en tant que composé de départ pour la préparation de taurolidine ou de taurultame au moyen de l'azide de 2-aminoéthanesulfonyle.
